# EUROPEAN PATENT SPECIFICATION

(11) **EP 0 792 165 B1**
(45) Date of publication and mention of the grant of the patent: **17.09.2003**
(21) Application number: 95936690.7
(22) Date of filing: 14.11.1995
(51) Int. Cl.: A61K 47/44, A61K 39/00

(54) **IMMUNOGENIC COMPOSITIONS**
IMMUNOGENE ZUBEREITUNGEN
COMPOSITIONS IMMUNOGENES

(30) Priority: 15.11.1994 GB 9422990
(43) Date of publication of application: 03.09.1997
(73) Proprietor: Vaxcine Limited, St. Helier, Jersey JE1 3UQ (GB)
(72) Inventor: NEW, Roger Randal Charles, Hampstead London NW3 6QB (GB)
(74) Representative: Chapman, Paul William
(86) International application number: GB9502675
(87) International publication number: WO96014871

(56) References cited:
- EP-A- 0 521 562
- WO-A-95/04524
- WO-A-95/13795
- US-A- 4 578 391
- US-A- 5 340 588
- RRC NEW: "Liposomes a practical approach", 1990, IRL PRESS,

## Description

The present invention relates to immunogenic compositions comprising an immunogen dissolved in a hydrophobic solvent in which it would not normally be soluble, to processes for preparing these compositions and to their use in the treatment or prophylaxis of disease, or use in the preparation of agents for such use. In particular, the invention relates to immunogenic compositions useful as oral vaccines.

For many applications, e.g in the pharmaceutical sciences, in food technology or the cosmetics industry, work with proteins and similar macromolecules presents problems because their hydrophilicity and high degree of polarity limit the extent to which they can interact with or incorporate into lipid phases. Many natural systems employ lipidic barriers (eg skin, cell membranes) to prevent access of hydrophilic molecules to internal compartments; the ability to disperse proteins in lipidic vehicles would open up a new route to introduction of these macromolecules into biological systems, whereby the lipid medium containing the protein can integrate with the hydrophobic constituents of barriers, instead of being excluded by them.

Another area where solubilisation of hydrophilic macromolecules in hydrophobic solvents would be useful is the area of compositions designed to elicit an immune response, e.g. vaccines, and in particular oral vaccines. Vaccines rely for their effectiveness in producing an immune response in a host to a specific antigen or group of antigens. Very often the antigenic component or components of a vaccine comprises one or more proteins, for example viral coat proteins. Administration of the vaccine to a subject induces antibody production in the subject leading to protection against infection by the particular agent from which the antigen or antigens were derived.

However, vaccines for oral administration which comprise proteins will have problems associated with them for the reasons discussed above. Thus, there exists a continuing need for oral vaccines which are capable of inducing the appropriate immune response when administered to a subject, but which are not subject to these problems.

It has now been found that such immune responses can be induced by the use of preparations where an immunogen is dissolved in a hydrophobic solvent such as an oil. In particular proteins or peptides solubilised in oils can be used to generate immune responses and therefore are useful in the preparation of vaccines, particularly oral vaccines.

Dispersion of hydrophilic substances in oil phase rather than aqueous media confers other benefits in terms of increasing their stability with respect to temperature-mediated denaturation, hydrolysis, light sensitivity etc. Oils can be chosen which remain fluid over a wider temperature range than aqueous solutions, or that have a higher viscosity, resulting in greater protection against physical damage. In mixed-phase systems, sequestration of proteins in oil can limit mutually harmful interactions - eg oxidation or hydrolysis - with watersoluble compounds.

There are examples of formulations containing both macromolecules and oil and one such example is disclosed in EP-A-0366277. The formulation disclosed in this document is an emulsion having both a hydrophobic and a hydrophilic phase, wherein the hydrophobic phase contains chylomicra or chylomicron-forming lipids. However, the macromolecule is dissolved in the hydrophilic phase not in the hydrophobic phase.

EP-A-0521994 also relates to a composition suitable for the oral delivery of macromolecules which comprises a biologically active material in association with lecithin or a compound capable of acting as a precursor for lecithin *in vivo*. All of the compositions exemplified are formulations which comprise a hydrophilic and a lipophilic phase. Once again, in this prior art document, the macromolecule is dissolved in the hydrophilic phase rather than in the lipophilic phase.

Although the formulations mentioned above do contain both macromolecules and oils, it is significant that in all cases the macromolecule is dissolved in the hydrophilic rather than in the lipophilic phase. Attempts to form true solutions of macromolecules in oils have met with limited success, and no examples of such solutions for use in vaccines are known.

US-A-5340588 discloses vaccine preparations based on lipospheres which are solid at room temperature. They have a layer of phospholipid embedded on the surface of a liposphere core and the antigen, or immunogen is either located in the core, can be attached to or within the phospholipid or both.

UK patent application No. 9323588.5 discloses a process by which a hydrophilic species can be solubilised in a hydrophobic solvent in which it would not normally be soluble. The process relies on the surprising discovery that if a hydrophilic species is mixed with an amphiphile under certain conditions, the resultant composition will be readily soluble in lipophilic solvents such as oils.

WO-A-9504524 relates to a composition comprising vesicles having a hydrophobic interior and loaded with biopolymeric substances such as antigens and vaccines.

EP-A-0521562 discloses dispersions of vesicles which have a non-polar interior in a non-polar phase.

US 5,340,588 relates to particles having a solid hydrophobic core containing an antigen and with a phospholipid embedded on the surface.

US 4,578,391 teaches a two phase emulsion composition which comprises lecithin, water and a hydrophobic drug.

The present invention relates to the surprising discovery that an immunogen solubilised in a lipophilic solvent such as an oil, can induce an immunogenic response in a subject to which it is administered. In particular, oral administration is facilitated.

This lipidic vehicle could also act as a more effective particulate delivery system, being readily taken up by phagocytosis.

Therefore, in a first aspect, the present invention provides a single phase essentially anhydrous immunogenic composition in which an immunogen is solubilised in a hydrophobic solvent in the absence of a hydrophilic phase, and comprising:
(i) an array of amphiphile molecules and immunogen moieties, wherein the amphiphile molecules are positioned around the immunogen moieties with their hydrophilic head groups orientated towards the immunogen moieties and wherein there is no chemical interaction between the amphiphile and the immunogen; and
(ii) a hydrophobic solvent around the amphiphile/immunogen array;
wherein the immunogen is selected from Diphtheria toxoid, tetanus toxoid, snake venom antigens, 'Hepatitis B antigens, whooping cough subunit, influenza a and/or b (or whole killed; virus or protein subunits), cholera antigens, *Helicobacter pylori* antigens, whole bacteria or extracts thereof, fungi or fungal antigens, polio virus, rotavirus, measles virus, rubella, respiratory syncitial virus, HIV, BCG, other mycobacterial antigens, *H. influenzae* A or B (with or without carrier protein), protozoal antigens, trematode antigens, cestode antigens, nematode antigens, surface membrane epitopes specific for cancer cells, and cell receptor targeting antiinflammatory immunomodulators, polymer conjugates of steroids; HLA antigens , pollens, dust mite antigens, bee stings or food allergens.

As used herein the term "immunogen relates to a species capable of eliciting an immune outcome. this outcome can be a typical immune response, e.g. production of antibodies, or the triggering of differentiation or expansion of specific populations of T cells, and can be systemic or local, e.g. restricted to a mucosal response. Alternatively, the immune outcome can be, for instance, immune tolerance, in which the naïve immune system is rendered unresponsive to challenge by a specific antigen.

Another alternative outcome may be desensitisation, in which a pre-existing tendency to an autoimmune or allergic response (IgE) against a specific antigen is reduced

Examples of suitable immunogens include Diphtheria toxoid, tetanus toxoid, botulin toxoid, snake venom antigens, Hepatitis B antigens, whooping cough subunit, influenza a and/or b (whole killed virus or protein subunits), cholera antigens, *H. pylori* antigens, whole bacteria, or extracts thereof, e.g. *P. aeruginosa, chlamydia* species, *neisseria* species, *yersinia* species, *salmonella* species, fungi or fungal antigens, e.g. from Candida, rabies virus, polio virus, rotavirus, measles virus, rubella, respiratory synctitial virus, HIV, BCG, other mycobacterial antigens, protozoal antigens, e.g. malaria, leishmania, toxoplasma, trypanosoma, trematode antigens, e.g. schistosoma, cestode antigens, e.g. from cysticerca, echinococcus, nematode antigens, e.g. toxocara, hookworm and filaria, spirochete antigens, e.g. *borrelia* species, surface membrane epitopes specific for cancer cells, and cell receptor targeting antiinflammatory immunomodulators (for treatment of inflammatory diseases such as Crohn's Disease and rheumatoid arthritis), e.g anti TNF-R and anti IL-1R.

Non-protein antigens may be used, such as, for example, polysaccharides or polymer conjugates of steroids.

One advantage of the present invention is that different antigens (e.g. proteins and polysaccharides) can be co-presented together in the same vehicle to elicit an enhanced immune response by virtue of one component acting as a carrier for the other, without the need for any covalent linkage.

Examples of antigens which can be used to reduce or eliminate an immune response against them include HLA antigens and polynucleotides such as RNA and DNA, including plasmid DNA.

Examples of antigens which can be used to elicit a desensitising immune response include pollens, dust mite antigens, bee stings and food allergens.

It is also possible, where the immunogen is a peptide, polysaccharide or other antigen, to conjugate it with at least one medium- or long-chain hydrocarbon tail.

In another embodiment, the immunogen is co-solubilised with one or more cytokines in order to enhance the response. Examples of suitable cytokines includeIL-4, IL-10, IL-12 and γ-interferons. Other immunostimmulants may also be incorporated, for example monophosphoryl lipid A, hydrobacterial extracts, muramyl dipeptide and analogues, tuftsin and cholera subunit B and heat labile toxin of *E. coli*.

In a second aspect, the invention provides a process for the preparation of a single phase essentially anhydrous immunogenic composition which comprises the following steps:
(i) either
   (a) mixing the immunogen with a dispersion of an amphiphile in a hydrophilic solvent, such that the amphiphile molecules form an assembly in which the hydrophilic head groups face outwards towards the hydrophilic phase which contains the immunogen, wherein the amphiphile assembly takes the form of micelles; or
   (b) mixing the immunogen with a dispersion of an amphiphile in a hydrophilic solvent, such that the amphiphile molecules form an assembly in which the hydrophilic head groups face outwards towards the hydrophilic phase which contains the immunogen, wherein the amphiphile assembly takes the form of small unilamellar vesicles; or
   (c) co-solubilizing the immunogen and an amphiphile in a common solvent;
(ii) removing the solvent or solvents to leave an array of amphiphile molecules and immunogen moieties, wherein the amphiphile molecules are positioned around the immunogen moieties with their hydrophilic head groups orientated towards the immunogen moieties and wherein there is no chemical interaction between the amphiphile and the immunogen; and
(iii) providing a hydrophobic solvent around the immunogen/amphiphile array;
wherein the immunogen is selected from Diphtheria toxoid, tetanus toxoid, snake venom antigens, Hepatitis B antigens, whooping cough subunit, influenza a and/or b (or whole killed virus or protein subunits), cholera antigens, *Helicobacter pylori* antigens, whole bacteria or extracts thereof, fungi or fungal antigens, polio virus, rotavirus, measles virus, rubella, respiratory syncitial virus, HIV, BCG, other mycobacterial antigens, *H. influenzae* A or B (with or without carrier protein), protozoal antigens, trematode antigens, cestode antigens, nematode antigens, surface membrane epitopes specific for cancer cells, and cell receptor targeting antiinflammatory immunomodulators, polymer conjugates of steroids; HLA antigens , pollens, dust mite antigens, bee stings or food allergens.

The processes disclosed in UK Patent Application No. 9323588.5 are particularly suitable for solubilising immunogens in hydrophobic solvents for use in the preparation of the compositions of the present invention.

There are numerous amphiphiles which may be used in the present invention and zwitterionic amphiphiles such as phospholipids are among those which have been found to be especially suitable. Phospholipids having a phosphatidyl choline head group have been used with particular success arid examples of such phospholipids include phosphatidyl choline (PC) itself, lyso-phosphatidyl choline (lyso-PC), sphingomylein, derivatives of any of these, for example hexadecylphosphocholine or amphiphilic polymers containing phosphoryl choline and halogenated amphiphiles e.g. fluorinated phospholipids. In the present application, the terms phosphatidyl choline (PC) and lecithin are used interchangeably. Suitable natural lecithins may be derived from any convenient source, for example egg and, in particular, soya. In most cases it is preferable to select an amphiphile which is chemically similar to the chosen hydrophobic solvent and this is discussed in greater detail below. Octyl glucosides, α-tocopherol, or esters thereof, or indeed mixtures of any of the above, can also be used.

The fact that the present inventors have found zwitterionic amphiphiles such as phospholipids to be particularly suitable for use in the process is a further indication of the significant differences between the present invention and the method of Okahata *et al*. Significantly, the authors of that prior art document concluded that anionic and zwitterionic lipids were completely unsuitable for use in their method and stated that they obtained zero yield of their complex using these lipids.

The hydrophobic solvent of choice will depend on the type of species to be solubilised and on the amphiphile. Suitable solvents include hydrocarbons, e.g. non-polar oils such as mineral oil, squalane and squalene, long chain fatty acids with unsaturated fatty acids such as oleic and linoleic acids being preferred, alcohols, particularly medium chain alcohols such as octanol and branched long chain alcohols such as phytol, isoprenoids, e.g. nerol, and geraniol, other alcohols such as t-butanol, terpineol, monoglycerides such as glycerol monooleate (GMO), other esters, e.g. ethyl acetate, amyl acetate and bornyl acetate, medium- or long-chain mono-, di- or tri-glycerides and mixtures thereof, halogenated analogues of any of the above including halogenated oils, e.g. long chain fluorocarbons and iodinated triglycerides, e.g. lipidiol.

Optimum results are generally obtained when the hydrophobic solvent and the amphiphile are appropriately matched. For example, with a solvent such as oleic acid, lyso-PC is a more suitable choice of amphiphile than PC, whereas the converse is true when the hydrophobic solvent is a triglyceride.

In addition, in some cases it has been found to be advantageous to add a quantity of the amphiphile to the hydrophobic solvent before it is brought into contact with the immunogen/amphiphile array. This ensures that the amphiphile molecules are not stripped away from their positions around the hydrophilic species because of the high affinity of the amphiphile for the hydrophobic solvent.

The orientation of amphiphile molecules into an array with their hydrophilic head groups facing the moieties of an immunogen can be achieved in several ways and examples of particularly suitable methods are discussed in more detail below.

In a first method, which has a similar starting point to the method described by Kirby *et al, in Bio*/*Technology*, November 1984, 979-984 and in *Liposome Technology*, Volume I, pages 19-27, Gregoriadis, Ed., CRC Press, Inc., Boca Raton, Florida, USA, an immunogen is mixed with a dispersion of an amphiphile in a hydrophilic solvent, such that the amphiphile molecules form an assembly in which the hydrophilic head groups face outwards towards the hydrophilic phase which contains the immunogen. The hydrophilic solvent is then removed to leave a dry composition in which the hydrophilic head groups of the amphiphile molecules are orientated towards the immunogen.

In this first method it is preferred that the hydrophilic solvent is water, although other polar solvents may be used.

The form taken by the amphiphile assembly may be micelles or small unilamellar vesicles which are generally understood to have a diameter of about 25 nm.

The weight ratio of amphiphile : immunogen will generally be in the region of from 1:1 to 100:1, preferably from 2:1 to 20:1 and most preferably about 8:1 for PC and 4:1 for lyso-PC.

These ratios are preferred ratios only and, in particular, it should be pointed out that the upper limit is set by economic considerations which mean that it is preferable to use the minimum possible amount of amphiphile. The lower limit is somewhat more critical and it is likely that ratios of 2:1 or below would only be used in cases where the immunogen has a significant hydrophobic portion or is exceptionally large.

Good performance is obtained when the solvent is removed quickly and a convenient method for the removal of the solvent is lyophilisation, although other methods can be used.

In some cases, it may be helpful to include salts in the hydrophilic solution, particularly if the immunogen is a macromolecular compound such as a large protein. However, because the presence of larger amounts of inorganic salts tends to give rise to the formation of crystals and, hence, to a cloudy solution, it is preferred that organic salts are used rather than inorganic salts such as sodium chloride. Ammonium acetate is especially suitable for this purpose since it has the additional advantage that it is easily removed by freeze drying.

A second method for the preparation of a composition containing an array of amphiphiles with their head groups pointing towards the moieties of the immunogen is to co-solubilise the immunogen and the amphiphile in a common solvent followed by removal of the solvent.

Therefore, in another preferred embodiment of the second aspect of the invention there is provided a process for preparing a vaccine which comprises co-solubilising an immunogen and an amphiphile in a common solvent and subsequently removing the common solvent thereby forming an immunogen/amphiphile array wherein the hydrophilic head groups of the amphiphile molecules are orientated towards the immunogen.

When this method is used, it is preferred that the weight ratio of amphiphile:immunogen is from about 1:1 to 50:1, preferably from 2:1 to 10:1 and most preferably about 4:1.

The common solvent must, of course, dissolve both the amphiphile and the immunogen and will, for preference, be a polar organic solvent such as dimethylformamide, dimethylsulphoxide or, most suitably, glacial acetic acid.

In this method, in contrast to the first method, it is unlikely that an array will be formed before the removal of the common solvent.

It seems probable that, on removal of the solvent, the amphiphile molecules tend to order themselves in sheets with their head groups towards the immunogen and their lipophilic tail groups facing away from the immunogen. However, the effectiveness of the present invention does not depend on the accuracy or otherwise of this observation.

It has been observed that good results are obtained when the solvent is removed slowly, for example by drying under a stream of nitrogen, probably because this allows more time for the amphiphile molecules to reorder themselves.

Any hydrophobic solvent for the amphiphile may be used, but for the water-in-oil emulsions preferred for use with small immunogens, a low boiling point solvent such as diethyl ether is preferred since it has been found that the best results are obtained when the hydrophobic solvent is removed slowly by gentle methods such as evaporation and, clearly, this is most effective using a low boiling point solvent. Low boiling point solvents are also preferred for water-in-oil emulsions although, for these, lyophilisation is a more suitable method of solvent removal. The hydrophilic solvent will preferably be aqueous.

The weight ratio of amphiphile:immunogen may be from about 1:1 to 50:1, preferably from 2:1 to 10:1 and most preferably about 4:1.

The ratio of hydrophilic solution to hydrophobic solution is not critical, but if small immunogens are used, it is preferably such as to ensure the formation of a water-in-oil emulsion rather than an oil-in-water emulsion.

An alternative method of forming the array, which may be particularly suited to use with small immunogens, is to entrap the immunogen in closed lipid vesicles such as small unilamellar vesicles (SUVs) dispersed in a hydrophilic solvent and then to remove the solvent.

One example of an amphiphile which is not capable of forming liposomes is lyso-lecithin. In most aqueous environments, this amphiphile forms micelles rather than small unilamellar vesicles and it is therefore unsuitable for use in the preparation of liposomes. It is however extremely useful in the process of the present invention, particularly when used in conjunction with a compatible hydrophobic solvent such as oleic acid.

In a third aspect, the present invention provides a single phase hydrophobic preparation of an immunogen in a hydrophobic solvent, obtainable using the methods described herein.

In a fourth aspect, the invention provides a two phase composition comprising a hydrophilic phase and a hydrophobic phase wherein the hydrophilic phase comprises an immunogenic composition of the invention. In particular, the hydrophobic phase is dispersed in a continuous hydrophilic phase, and is preferably an emulsion.

In a fifth aspect the present invention provides the use of an immunogen dissolved in a hydrophobic solvent in which it would not normally be soluble in the preparation of an immunogenic composition, particularly an oral vaccine.

In a sixth aspect, the invention provides an enteric coated capsule containing an immunogenic composition or a single phase hydrophobic preparation of the invention.

One advantage of the single phase preparations described above is that they are essentially anhydrous and therefore stable to hydrolysis. They are also stable to freeze-thawing and have greater stability at high temperatures, probably because water must be present in order for the protein to unfold and become denatured. This means that they may be expected to have a much longer shelf life than aqueous preparations of the immunogens.

The invention will now be further described with reference to the following examples, which should not be construed as limiting the invention.

The examples refer to the figures in which:
FIGURE 1: shows response to tetanus toxoid after oral administration of a formulation of the invention; and
FIGURE 2: shows response to tetanus toxoid after subcutaneous administration of a formulation of the invention.

### EXAMPLE 1

1 ml of tetanus toxoid, at a concentration of 3000 Lf/ml (6 mg/ml) was dialysed overnight against 1 litre of distilled water. Soya phosphatidyl choline was dispersed in distilled water by probe sonication for 10 minutes with cooling at a concentration of 100 mg/ml. 1 ml of this solution was dispensed into a glass screw-capped 2 ml vial, and 40 µl of tetanus toxoid (5 mg/ml after dilution) was added with mixing. The mixture was lyophilised overnight, and 1 ml of oleic acid added. A crystal clear solution was obtained, which was stored frozen until required for use.

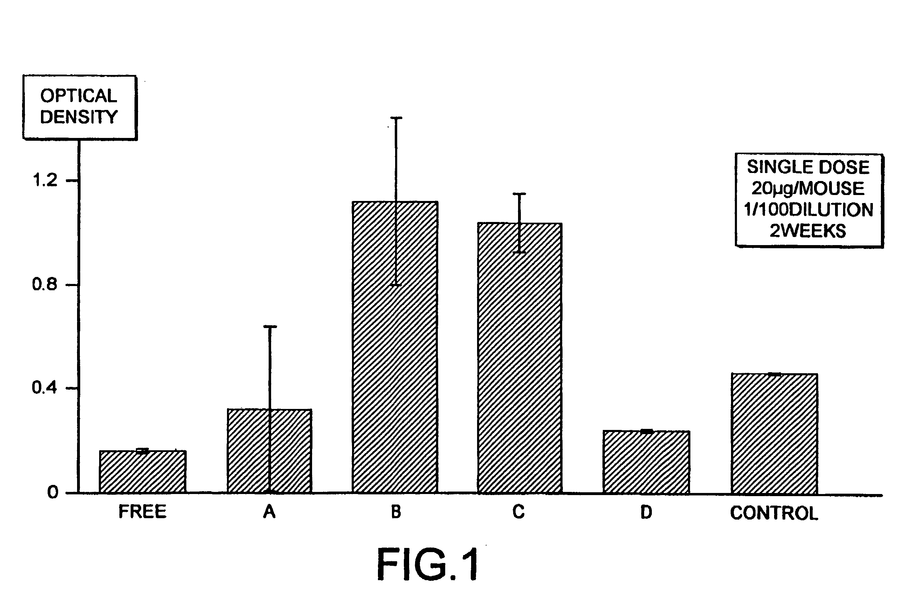

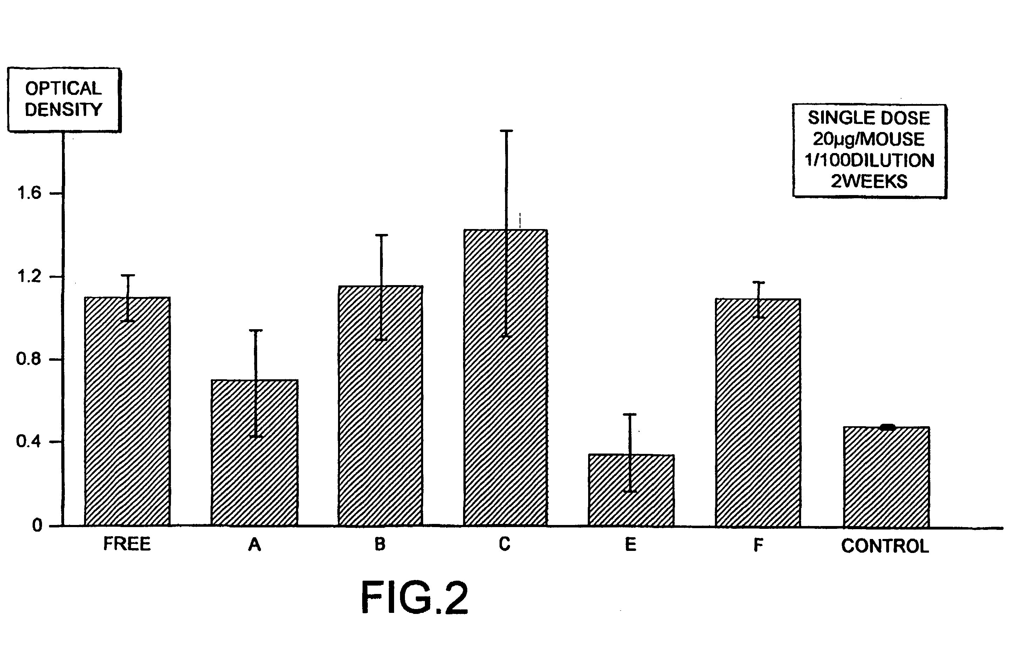

100 µl of this preparation (referred to as formulation 'A') was administered either subcutaneously or through an intragastric tube to inbred young adult Swiss mice (20 µg tetanus toxoid per animal, 3-4 mice per group) housed three to a cage under controlled conditions and fed with food and water *ad lib*. Plasma samples were taken two weeks after administration from the tail vein, and the antibody (IgG) levels against tetanus antigen measured by sandwich ELISA at a 1:100 dilution. The results, expressed as optical density at 450 nm, are shown in figures 1 and 2.

### EXAMPLE 2

Soya phosphatidyl choline was dispersed in distilled water by probe sonication for 10 minutes, with cooling, at a concentration of 100 mg/ml. 1 ml of this solution was dispensed into a glass screw-capped 2 ml vial, and 40 µl of tetanus toxoid, as in Example 'A', was added with mixing. The mixture was lyophilised overnight, and 1 ml of Miglyol 818 was added. A crystal clear solution was obtained, which was stored frozen until required for use.

100 µl of this preparation (referred to as formulation 'B') was administered either subcutaneously or through an intragastric tube to inbred young adult Swiss mice (20 µg tetanus toxoid per animal, 3-4 mice per group) housed three to a cage under controlled conditions and fed with food and water *ad lib*. Plasma samples were taken two weeks after administration from the tail vein, and the antibody (IgG) levels measured by sandwich ELISA at a 1:100 dilution. The results, expressed as optical density at 450 nm, are shown in figures 1 and 2.

### EXAMPLE 3

Soya phosphatidyl choline was dispersed in distilled water by probe sonication for 10 minutes with cooling at a concentration of 100 mg/ml. 1 ml of this solution was dispensed into a glass screw-capped 2 ml vial, and 40 µl of tetanus toxoid, as in Example 'A', was added with mixing. The mixture was lyophilised overnight, and 1 ml of a commercial source of sunflower oil was added. A crystal clear solution was obtained, which was stored frozen until required for use.

100 µl of this preparation (referred to as formulation 'C') was administered either subcutaneously or through an intragastric tube to inbred young adult Swiss mice (20 µg tetanus toxoid per animal, 3-4 mice per group) housed three to a cage under controlled conditions and fed with food and water *ad lib*. Plasma samples were taken two weeks after administration from the tail vein, and the antibody (IgG) levels measured by sandwich ELISA at a 1:100 dilution. The results, expressed as optical density at 450 nm, are shown in figures 1 and 2.

### EXAMPLE 4

Hexadecyl phosphoryl choline was dissolved in distilled water at a concentration of 100 mg/ml. 500 µl of this solution was dispensed into a glass screw-capped 2 ml vial, and 20 µl of tetanus toxoid, as in Example 'A', (5 mg/ml after dilution) was added with mixing. The mixture was lyophilised overnight, and 500 µl of oleic acid added. A crystal clear solution was obtained, which was stored frozen until required for use.

100 µl of this preparation (referred to as formulation 'D') was administered through an intragastric tube to inbred young adult Swiss mice (20 µg tetanus toxoid per animal, 3-4 mice per group) housed three to a cage under controlled conditions and fed with food and water *ad lib*. Plasma samples were taken two weeks after administration from the tail vein, and the antibody (IgG) levels measured by sandwich ELISA at a 1:100 dilution. The results, expressed as optical density at 450 nm, are shown in figure 1.

### EXAMPLE 5

β-octyl glucoside was dissolved in distilled water at a concentration of 100 mg/ml. 500 µl of this solution was dispensed into a glass screw-capped 2 ml vial, and 20 µl of tetanus toxoid, as in Example 'A', (5 mg/ml after dilution) was added with mixing. The mixture was lyophilised overnight, and 500 µl of glycerol monooleate was added. A crystal clear solution was obtained, which was stored frozen until required for use.

100 µl of this preparation (referred to as formulation 'E') was administered subcutaneously to inbred young adult Swiss mice (20 µg tetanus toxoid per animal, 3-4 mice per group) housed three to a cage under controlled conditions and fed with food and water *ad lib*. Plasma samples were taken two weeks after administration from the tail vein, and the antibody (IgG) levels measured by sandwich ELISA at a 1:100 dilution. The results, expressed as optical density at 450 nm, are shown in figure 2.

### EXAMPLE 6

The amphiphile β-octyl glucoside was dissolved in distilled water at a concentration of 100 mg/ml. 500 µl of this solution was dispensed into a glass screw-capped 2 ml vial, and 20 µl of tetanus toxoid, as in Example 'A', (5 mg/ml after dilution) was added with mixing. The mixture was lyophilised overnight, and 500 µl of phytol was added. A crystal clear solution was obtained, which was stored frozen until required for use.

100 µl of this preparation (referred to as formulation 'F') was administered subcutaneously to inbred young adult Swiss mice (20 µg tetanus toxoid per animal, 3-4 mice per group) housed three to a cage under controlled conditions and fed with food and water *ad lib*. Plasma samples were taken two weeks after administration from the tail vein, and the antibody (IgG) levels measured by sandwich ELISA at a 1:100 dilution. The results, expressed as optical density at 450 nm, are shown in figure 2.

## Claims

1. A single phase essentially anhydrous immunogenic composition in which an immunogen is solubilised in a hydrophobic solvent in the absence of a hydrophilic phase, arid comprising:
(i) an array of amphiphile molecules and immunogen moieties, wherein the amphiphile molecules are positioned around the immunogen moieties with their hydrophilic head groups orientated towards the immunogen moieties and wherein there is no chemical interaction between the amphiphile and the immunogen; and
(ii) a hydrophobic solvent around the amphiphile/immunogen array;
wherein the immunogen is selected from Diphtheria toxoid, tetanus toxoid, snake venom antigens, Hepatitis B antigens, whooping cough subunit, influenza a and/or b (or whole killed virus or protein subunits), cholera antigens, *Helicobacter pylori* antigens, whole bacteria or extracts thereof, fungi or fungal antigens, polio virus, rotavirus, measles virus, rubella, respiratory syncitial virus, HIV, BCG, other mycobacterial antigens, *H. influenzae* A or B (with or without carrier protein), protozoal antigens, trematode antigens, cestode antigens, nematode antigens, surface membrane epitopes specific for cancer cells, and cell receptor targeting antiinflammatory immunomodulators, polymer conjugates of steroids; HLA antigens , pollens, dust mite antigens, bee stings or food allergens.

2. An immunogenic composition as claimed in claim 1, wherein the immunogen is an antigen.

3. An immunogenic composition as claimed in or claim 1 or claim 2 which is a vaccine or can be used in the preparation of a vaccine.

4. An immunogenic composition as claimed in claim 3 wherein the vaccine is an oral vaccine.

5. An immunogenic composition as claimed in any one of claims 1 to 4 wherein two or more antigens are co-presented in the composition.

6. An immunogenic composition as claimed in claim 5 wherein a protein antigen and a polysaccharide antigen are co-presented.

7. An immunogenic composition as claimed in any one of claims 1 to 6 wherein one or more additional immunostimulants is present.

8. An immunogenic composition as claimed in claim 7 wherein the additional immunostimmulant(s) is/are selected from IL-4, IL-10, IL-12, γ-interferons, monophosphoryl lipid A, muramyl dipeptide and analogues thereof, tuftsin and cholera subunit B.

9. An immunogenic composition as claimed in claim 1 which will cause a reduction or an elimination of immune response against the antigen.

10. An immunogenic composition as claimed in claim 9 wherein the antigen is an HLA antigen.

11. An immunogenic composition as claimed in claim 1 wherein the composition will desensitise the immune response.

12. An immunogenic composition as claimed in claim 11 wherein the antigen is selected from pollens, dust mite antigens or food allergens.

13. A process for the preparation of a single phase essentially anhydrous immunogenic composition which comprises the following steps:
(i) either
(a) mixing the immunogen with a dispersion of an amphiphile in a hydrophilic solvent, such that the amphiphile molecules form an assembly in which the hydrophilic head groups face outwards towards the hydrophilic phase which contains the immunogen, wherein the amphiphile assembly takes the form of micelles; or
(b) mixing the immunogen with a dispersion of an amphiphile in a hydrophilic solvent, such that the amphiphile molecules form an assembly in which the hydrophilic head groups face outwards towards the hydrophilic phase which contains the immunogen, wherein the amphiphile assembly takes the form of small unilamellar vesicles; or
(c) co-solubilizing the immunogen and an amphiphile in a common solvent;
(ii) removing the solvent or solvents to leave an array of amphiphile molecules and immunogen moieties, wherein the amphiphile molecules are positioned around the immunogen moieties with their hydrophilic head groups orientated towards the immunogen moieties and wherein there is no chemical interaction between the amphiphile and the immunogen; and
(iii) providing a hydrophobic solvent around the immunogen/amphiphile array;
wherein the immunogen is selected from Diphtheria toxoid, tetanus toxoid, snake venom antigens, Hepatitis B antigens, whooping cough subunit, influenza a and/or b (or whole killed virus or protein subunits), cholera antigens, *Helicobacter pylori* antigens, whole bacteria or extracts thereof, fungi or fungal antigens, polio virus, rotavirus, measles virus, rubella, respiratory syncitial virus, HIV, BCG, other mycobacterial antigens, *H. influenzae* A or B (with or without carrier protein), protozoal antigens, trematode antigens, cestode antigens, nematode antigens, surface membrane epitopes specific for cancer cells, and cell receptor targeting antiinflammatory immunomodulators, polymer conjugates of steroids; HLA antigens , pollens, dust mite antigens, bee stings or food allergens.

14. A process as claimed in claim 13 for the preparation of a composition as claimed in any one of claims 1 to 12.

15. A process as claimed in claim 13 or claim 14, wherein the amphiphile is a phospholipid.

16. A process as claimed in claim 15 wherein the amphiphile is a phospholipid with a phosphatidyl choline head group.

17. A process as claimed in claim 16, wherein the phospholipid is phosphatidyl choline (PC), lyso-phosphatidyl choline (lyso-PC), sphingomyelin, a derivative of one of the above such as hexadecyl phosphatidyl choline or an amphiphile polymer containing phosphoryl choline.

18. A process as claimed in any one of claims 13 to 17, wherein the hydrophobic solvent comprises a hydrocarbon (eg mineral oil or squalene), a long chain fatty acid, a medium chain alcohol or polyol eg α-tocopherol, a medium or long chain mono-, di- or tri-glyceride or mixtures thereof.

19. A process as claimed in any one of claims 13 to 18, wherein the amphiphile comprises PC and the hydrophobic solvent is a triglyceride or wherein the amphiphile comprises lyso-PC and the hydrophobic solvent is oleic acid.

20. A process as claimed in any one of claims 13 to 19, wherein the process includes step i(a) or i(b) and wherein the hydrophilic solvent is removed by lyophilisation.

21. A process as claimed in any one of claims 13 to 20, wherein the process includes step i(a) or i(b) and wherein the hydrophilic solvent is water.

22. A process as claimed in any one of claims 13 to 21, wherein the process includes step i(c) and wherein the common solvent is dimethyl formamide, dimethyl sulphoxide or glacial acetic acid.

23. A process as claimed in any one of claims 13 to 19 or 22, which includes step i(c) and wherein the weight ratio of amphiphile to hydrophilic species is from about 1:1 to 50:1.

24. A single phase hydrophobic preparation of an immunogen in a hydrophobic solvent obtainable by a process as claimed in any one of claims 13 to 23.

25. A two phase composition comprising a hydrophilic phase and a hydrophobic phase, wherein the hydrophobic phase comprises a preparation as claimed in any one of claims 1 to 12 or 24.

26. A composition as claimed in claim 25, wherein the hydrophobic phase is dispersed in a continuous hydrophilic phase.

27. A composition as claimed in claim 25 or claim 26 which is an emulsion.

28. An enteric coated capsule containing an immunogenic composition as defined in any one of claims 1 to 12 or a hydrophobic preparation as defined in claim 24.

## Patentansprüche

1. Einphasige, im wesentlichen wasserfreie Immunogen- Zusammensetzung, in der ein Immunogen in einem hydrophoben Lösemittel in Abwesenheit einer hydrophilen Phase solubilisiert wird, wobei die Zusammensetzung aufweist:
(i) eine Gruppe aus amphiphilen Molekülen und Immunogen-Komponenten, wobei die amphiphilen Moleküle rund um die Immunogen-Komponenten angeordnet sind, wobei deren hydrophile Kopfgruppen zu den Immunogen-Komponenten hin ausgerichtet sind, und wobei keine chemische Interaktion zwischen den amphiphilen Molekülen und den Immunogen-Komponenten stattfindet; und
(ii) ein hydrophobes Lösemittel rund um die Gruppe aus amphiphilen Molekülen und Immunogen-Komponenten;
wobei das Immunogen ausgewählt wird aus Diphterie-Toxoid, Tetanus-Toxoid, Schlangengift-Antigenen, Hepatitis-B-Antigenen, Keuchhusten-Untereinheiten, Influenza a und/oder b (oder ganze getötete Virus- oder Protein-Untereinheiten), Cholera-Antigenen, *Helicobacter pylori* Antigenen, ganzen Bakterien oder Extrakten davon, Pilzen oder Pilz-Antigenen, Polio-Virus, Rotavirus, Masernvirus, Röteln, Respiratory-syncytial-Virus, HIV, BCG, anderen mykobakteriellen Antigenen, *H. influenzae* A oder B (mit oder ohne Trägerprotein), Prozoon-Antigenen, Trematoden-Antigenen, Zestoden-Antigenen, Nematoden-Antigenen, für Krebszellen spezifischen Oberflächenmembranenepitopen, und entzündungshemmenden Immunmodulatoren, die auf Zellrezeptoren abzielen, Polymerkonjugaten von Steroiden; HLA-Antigenen, Pollen, Staubmilben-Antigenen, Bienenstich- oder Lebensmittelallergenen.

2. Immunogene Zusammensetzung nach Anspruch 1, wobei es sich bei dem Immunogen um ein Antigen handelt.

3. Immunogene Zusammensetzung nach Anspruch 1 oder Anspruch 2, wobei es sich um ein Vakzin handelt oder diese Zusammensetzung bei der Herstellung eines Vakzins verwendet werden kann.

4. Immunogene Zusammensetzung nach Anspruch 3, wobei es sich bei dem Vakzin um ein orales Vakzin handelt.

5. Immunogene Zusammensetzung nach einem der Ansprüche 1 bis 4, wobei zwei oder mehr Antigene in der Zusammensetzung gemeinsam vorhanden sind.

6. Immunogene Zusammensetzung nach Anspruch 5, wobei ein Protein-Antigen und ein Polysaccharid-Antigen gemeinsam vorhanden sind.

7. Immunogene Zusammensetzung nach einem der Ansprüche 1 bis 6, wobei ein oder mehrere zusätzliche das Immunsystem stimulierende Substanzen vorhanden sind.

8. Immunogene Zusammensetzung nach Anspruch 7, wobei die zusätzliche(n) das Immunsystem stimulierende(n) Substanz(en) ausgewählt wird/werden aus IL-4, IL-10, IL-12, γ-Interferonen, Monophosphoryl-Lipid A, Muramyl-Dipeptid und Analogen davon, Tuftsin und Cholera-Untereinheit B.

9. Immunogene Zusammensetzung nach Anspruch 1, welche eine Verringerung oder Beseitigung der Immunreaktion auf das Antigen verursacht.

10. Immunogene Zusammensetzung nach Anspruch 9, wobei es sich bei dem Antigen um ein HLA-Antigen handelt.

11. Immunogene Zusammensetzung nach Anspruch 1, wobei die Zusammensetzung die Ansprechempfindlichkeit der Immunreaktion reduziert.

12. Immunogene Zusammensetzung nach Anspruch 11, wobei das Antigen ausgewählt wird aus Pollen, Staubmilben-Antigenen oder Lebensmittel-Allergenen.

13. Verfahren zur Herstellung einer einphasigen, im wesentlichen wasserfreien immunogenen Zusammensetzung, welche die folgenden Schritte aufweist:
(i) entweder
(a) das Mischen des Immunogens mit einer Dispersion eines Amphiphils in einem hydrophilen Lösemittel, so dass die amphiphilen Moleküle eine Gruppe bilden, in der die hydrophilen Kopfgruppen nach außen zur hydrophilen Phase gerichtet sind, welche das Immunogen enthält, wobei die amphiphile Gruppe die Form von Mizellen annimmt; oder
(b) das Mischen des Immunogens mit einer Dispersion eines Amphiphils in einem hydrophilen Lösemittel, so dass die amphiphilen Moleküle eine Gruppe bilden, in der die hydrophilen Kopfgruppen nach außen zur hydrophilen Phase gerichtet sind, welche das Immunogen enthält, wobei die amphiphile Gruppe die Form kleiner, unilamellarer Bläschen annimmt; oder
(c) das gemeinsame Lösen des Immunogens und eines Amphiphils in einem gemeinsamen Lösemittel;
(ii) das Entfernen des Lösemittels oder der Lösemittel, um eine Gruppe amphiphiler Moleküle und immunogener Komponenten zu hinterlassen, wobei die amphiphilen Moleküle rund um die immunogenen Komponenten angeordnet werden, wobei ihre hydrophilen Kopfgruppen zu den immunogenen Komponenten hin gerichtet sind, und wobei keine chemische Interaktion zwischen den Amphiphilen und den Immunogenen stattfindet; und
(iii) das Bereitstellen eines hydrophoben Lösemittels rund um die Immunogen/Amphiphilen-Gruppe;
wobei das Immunogen ausgewählt wird aus Diphterie-Toxoid, Tetanus-Toxoid, Schlangengift-Antigenen, Hepatitis-B-Antigenen, Keuchhusten-Untereinheiten, Influenza a und/oder b (oder ganze getötete Virus- oder Protein-Untereinheiten), Cholera-Antigenen, *Helicobacter pylori* Antigenen, ganzen Bakterien oder Extrakten davon, Pilzen oder Pilz-Antigenen, Polio-Virus, Rotavirus, Masernvirus, Röteln, Respiratory-syncytial-Virus, HIV, BCG, anderen mykobakteriellen Antigenen, *H. influenzae* A oder B (mit oder ohne Trägerprotein), Prozoon-Antigenen, Trematoden-Antigenen, Zestoden-Antigenen, Nematoden-Antigenen, für Krebszellen spezifische Oberflächenmembranenepitopen, und entzündungshemmenden Immunmodulatoren, die auf Zellrezeptoren abzielen, Polymerkonjugaten von Steroiden; HLA-Antigenen, Pollen, Staubmilben-Antigenen, Bienenstich- oder Lebensmittelallergenen.

14. Verfahren nach Anspruch 13 für die Herstellung einer Zusammensetzung nach einem der Ansprüche 1 bis 12.

15. Verfahren nach Anspruch 13 oder 14, wobei es sich bei dem Amphiphil um ein Phospholipid handelt.

16. Verfahren nach Anspruch 15, wobei es sich bei dem Amphiphil um ein Phospholipid mit einer Phosphatidylcholin-Kopfgruppe handelt.

17. Verfahren nach Anspruch 16, wobei es sich bei dem Phospholipid um Phosphatidylcholin (PC), Lyso-phosphatidylcholin (Lyso-PC), Sphingomyelin, einem Derivat von einer der obigen Substanzen, wie zum Beispiel Hexadecylphosphatidylcholin oder einem amphiphilen Polymer handelt, das Phosphorylcholin enthält.

18. Verfahren nach einem der Ansprüche 13 bis 17, wobei das hydrophobe Lösungsmittel einen Kohlenwasserstoff (z.B. Mineralöl oder Squalen), eine langkettige Fettsäure, einen mittelkettigen Alkohol oder ein Polyol, z.B. α-Tocopherol, ein mittel- oder langkettiges Mono-, Di- oder Triglycerid oder Mischungen davon aufweist.

19. Verfahren nach einem der Ansprüche 13 bis 18, wobei das Amphiphil PC aufweist, und wobei es sich bei dem hydrophoben Lösemittel um ein Triglycerid handelt, oder wobei das Amphiphil Lyso-PC enthält und es sich bei dem hydrophoben Lösemittel um Oleinsäure handelt.

20. Verfahren nach einem der Ansprüche 13 bis 19, wobei das Verfahren den Schritt i(a) oder i(b) aufweist, und wobei das hydrophile Lösemittel durch Lyophilisierung entfernt wird.

21. Verfahren nach einem der Ansprüche 13 bis 20, wobei das Verfahren den Schritt i(a) oder i(b) aufweist, und wobei es sich bei dem hydrophilen Lösemittel um Wasser handelt.

22. Verfahren nach einem der Ansprüche 13 bis 21, wobei das Verfahren den Schritt i(c) aufweist und wobei es sich bei dem gemeinsamen Lösemittel um Dimethylformamid, Dimethylsulphoxid oder Eisessig handelt.

23. Verfahren nach einem der Ansprüche 13 bis 19 oder 22, welches den Schritt i(c) aufweist und wobei das Gewichtsverhältnis zwischen der amphiphilen und hydrophilen Art etwa 1:1 bis 50:1 beträgt.

24. Einphasige hydrophobe Zubereitung eines Immunogens in einem hydrophoben Lösemittel, welche durch ein Verfahren nach einem der Ansprüche 13 bis 23 erhaltbar ist.

25. Zweiphasige Zusammensetzung, welche eine hydrophile Phase und eine hydrophobe Phase aufweist, wobei die hydrophobe Phase eine Zubereitung nach einem der Ansprüche 1 bis 12 oder 24 aufweist.

26. Zusammensetzung nach Anspruch 25, wobei die hydrophobe Phase in einer kontinuierlichen hydrophilen Phase dispergiert ist.

27. Zusammensetzung nach Anspruch 25 oder Anspruch 26, welche eine Emulsion ist.

28. Von einer enterischen Schutzhülle umgebene Kapsel, die eine immunogene Zusammensetzung nach einem der Ansprüche 1 bis 12 oder eine hydrophobe Zubereitung nach Anspruch 24 enthält.

## Revendications

1. Composition immunogène essentiellement en phase unique dans laquelle un immunogène est solubilisé dans un solvant hydrophobe en l'absence d'une phase hydrophile, et comprenant :
(i) une configuration de molécules amphiphiles et de parties immunogènes, dans laquelle les molécules amphiphiles sont disposées autour des parties immunogènes avec leurs groupements de tête hydrophile orientés vers les parties immunogènes et dans laquelle il n'y a aucune interaction chimique entre l'amphiphile et l'immunogène ; et
(ii) un solvant hydrophobe autour de la configuration amphiphile/immunogène ;
dans laquelle l'immunogène est choisi parmi l'anatoxine diphtérique, l'anatoxine tétanique, des antigènes de venin de serpent, des antigènes de l'hépatite B, une sous-unité de la coqueluche, le virus de la grippe a et/ou b (ou un virus entier tué ou des sous-unités de protéine), des antigènes du choléra, des antigènes d'*Helicobacter pylori*, la bactérie entière ou des extraits de celle-ci, des antigènes de champignons ou fongiques, le virus de la polio, le rotavirus, le virus de la rougeole, le virus de la rubéole, le virus respiratoire syncytial, le HIV, le BCG, d'autres antigènes mycobactériens, *H. influenza* A ou B (avec ou sans protéine support), des antigènes de protozoaires, des antigènes de trématodes, des antigènes de cestodes, des antigènes de nématodes, des épitopes de membrane de surface spécifiques à des cellules cancéreuses, et des immunomodulateurs anti-inflammatoires ciblant le récepteur cellulaire, des conjugués polymères de stéroïdes ; des antigènes HLA, des pollens, des antigènes d'acariens de la poussière, des allergènes de piqûre d'abeille ou d'aliments.

2. Composition immunogène selon la revendication 1 dans laquelle l'immunogène est un antigène.

3. Composition immunogène selon la revendication 1 ou la revendication 2 qui est un vaccin ou peut être utilisée dans la préparation d'un vaccin.

4. Composition immunogène selon la revendication 3 dans laquelle le vaccin est un vaccin oral.

5. Composition immunogène selon l'une quelconque des revendications 1 à 4 dans laquelle deux antigènes ou plus sont présents simultanément dans la composition.

6. Composition immunogène selon la revendication 5 dans laquelle un antigène de protéine et un antigène de polysaccharide sont présents simultanément.

7. Composition immunogène selon l'une quelconque des revendications 1 à 6 dans laquelle un ou plusieurs immunostimulants supplémentaires sont présents.

8. Composition immunogène selon la revendication 7 dans laquelle le(s) immunostimulant(s) supplémentaire(s) est/sont choisi(s) parmi l'IL-4, l'IL-10, l'IL-12, les γ-interférons, le monophosphoryllipide A, le muramyldipeptide et les analogues de celui-ci, la tuftsine et la sous-unité B du choléra.

9. Composition immunogène selon la revendication 1 qui provoquera une réduction ou une élimination de la réponse immune contre l'antigène.

10. Composition immunogène selon la revendication 9 dans laquelle l'antigène est un antigène HLA.

11. Composition immunogène selon la revendication 1 dans laquelle la composition désensibilisera la réponse immune.

12. Composition immunogène selon la revendication 11 dans laquelle l'antigène est choisi parmi les pollens, les antigènes des acariens de la poussière ou des allergènes d'aliments.

13. Procédé pour la préparation d'une composition immunogène essentiellement en phase unique qui comprend les étapes suivantes :
(i) soit
(a) le mélange de l'immunogène avec une dispersion d'un amphiphile dans un solvant hydrophile, de sorte que les molécules amphiphiles forment un assemblage dans lequel les groupements de tête hydrophile font face vers l'extérieur à la phase hydrophile qui contient l'immunogène, dans lequel l'assemblage amphiphile prend la forme de micelles ; ou
(b) le mélange de l'immunogène avec une dispersion d'un amphiphile dans un solvant hydrophile, de sorte que les molécules amphiphiles forment un assemblage dans lequel les groupements de tête hydrophile font face vers l'extérieur à la phase hydrophile qui contient l'immunogène, dans lequel l'assemblage amphiphile prend la forme de petites vésicules unilamellaires ; ou
(c) la solubilisation simultanée de l'immunogène et d'un amphiphile dans un solvant commun ;
(ii) l'élimination du ou des solvant(s) pour laisser une configuration des molécules amphiphiles et des parties immunogènes, dans laquelle les molécules amphiphiles sont disposées autour des parties immunogènes avec leurs groupements de tête hydrophile orientés vers les parties immunogènes et dans laquelle il n'y a aucune interaction chimique entre l'amphiphile et l'immunogène ; et
(iii) la fourniture d'un solvant hydrophobe autour de la configuration immunogène/amphiphile ;
dans lequel l'immunogène est choisi parmi l'anatoxine diphtérique, l'anatoxine tétanique, des antigènes de venin de serpent, des antigènes de l'hépatite B, une sous-unité de la coqueluche, le virus de la grippe a et/ou b (ou un virus entier tué ou des sous-unités de protéine), des antigènes du choléra, des antigènes d'*Helicobacter pylori*, la bactérie entière ou des extraits de celle-ci, des antigènes de champignons ou fongiques, le virus de la polio, le rotavirus, le virus de la rougeole, le virus de la rubéole, le virus respiratoire syncytial, le HIV, le BCG, d'autres antigènes mycobactériens, *H. influenza* A ou B (avec ou sans protéine support), des antigènes de protozoaires, des antigènes de trématodes, des antigènes de cestodes, des antigènes de nématodes, des épitopes de membrane de surface spécifiques de cellules cancéreuses, et des immunomodulateurs anti-inflammatoires ciblant le récepteur cellulaire, des conjugués polymères de stéroides ; des antigènes HLA, des pollens, des antigènes d'acariens de la poussière, des allergènes de piqûre d'abeille ou d'aliments.

14. Procédé selon la revendication 13 pour la préparation d'une composition selon l'une quelconque des revendications 1 à 12.

15. Procédé selon la revendication 13 ou la revendication 14 dans lequel l'amphiphile est un phospholipide.

16. Procédé selon la revendication 15 dans lequel l'amphiphile est un phospholipide avec un groupement de tête de phosphatidylcholine.

17. Procédé selon la revendication 16 dans lequel le phospholipide est la phosphatidylcholine (PC), la lysophosphatidylcholine (lyso-PC), la sphingomyéline, un dérivé de l'une de celles-ci comme l'hexadécylphosphatidylcholine ou un polymère amphiphile contenant de la phosphorylcholine.

18. Procédé selon l'une quelconque des revendications 13 à 17 dans lequel le solvant hydrophobe comprend un hydrocarbone (par exemple, de l'huile de vaseline ou du squalène), un acide gras à chaîne longue, un alcool ou polyol à chaîne moyenne par exemple l'α-tocophérol, un mono-, di- ou triglycéride à chaîne moyenne ou longue ou des mélanges de ceux-ci.

19. Procédé selon l'une quelconque des revendications 13 à 18 dans lequel l'amphiphile comprend de la PC et le solvant hydrophobe est un triglycéride ou dans lequel l'amphiphile comprend de la lyso-PC et le solvant hydrophobe est l'acide oléique.

20. Procédé selon l'une quelconque des revendications 13 à 19 dans lequel le procédé inclut l'étape i(a) ou i(b) et dans lequel le solvant hydrophile est éliminé par lyophilisation.

21. Procédé selon l'une quelconque des revendications 13 à 20 dans lequel le procédé inclut l'étape i(a) ou i(b) et dans lequel le solvant hydrophile est l'eau.

22. Procédé selon l'une quelconque des revendications 13 à 21 dans lequel le procédé inclut l'étape i(c) et dans lequel le solvant commun est le diméthylformamide, le diméthylsulfoxyde ou l'acide acétique glacial.

23. Procédé selon l'une quelconque des revendications 13 à 19 ou 22 qui inclut l'étape i(c) et dans lequel le rapport en poids de l'espèce amphiphile à l'espèce hydrophile est d'environ 1:1 à 50:1.

24. Préparation hydrophobe en phase unique d'un immunogène dans un solvant hydrophobe susceptible d'être obtenue par un procédé selon l'une quelconque des revendications 13 à 23.

25. Composition à deux phases comprenant une phase hydrophile et une phase hydrophobe dans laquelle la phase hydrophobe comprend une préparation selon l'une quelconque des revendications 1 à 12 ou 24.

26. Composition selon la revendication 25 dans laquelle la phase hydrophobe est dispersée dans une phase hydrophile continue.

27. Composition selon la revendication 25 ou la revendication 26 qui est une émulsion.

28. Capsule à délitage entérique contenant une composition immunogène comme définie dans l'une quelconque des revendications 1 à 12 ou une préparation hydrophobe comme définie dans la revendication 24.
